# EUROPEAN PATENT APPLICATION

(11) **EP 2 008 587 A1**
(43) Date of publication of application: **31.12.2008**
(21) Application number: 07740817.7
(22) Date of filing: 02.04.2007
(51) Int. Cl.: A61B 5/151

(54) **LANCET ASSEMBLY**

(30) Priority: 03.04.2006 JP 2006102165
(71) Applicant: Izumi-Cosmo Company Limited, Osaka-shi, Osaka 530-0005 (JP)
(72) Inventor: KITAMURA, Yoritaka, Tokyo 103-0022 (JP); ABE, Teruyuki, Tokyo 103-0022 (JP); SEKI, Kazuharu, Tokyo 1540001 (JP)
(74) Representative: HOFFMANN EITLE
(86) International application number: PCT/JP2007/057381
(87) International publication number: WO 2007/114423

(57) **Abstract**

In a lancet assembly 100 including a lancet 200 which includes lancet 200 including a lancet body 204, a lancet cap 206 and a pricking member 210 made of a metal as well as a lancet case 102 that houses a portion of the lancet, the lancet body has a protruding portion 212, the lancet case has a case body 114 and wings 116 which are present on the both sides of the lancet case, each wing has a stopper 122 that protrudes inward between its front end portion 118 and its rear end portion 120, the front end portion is connected to the case body and the rear end portion is left free, and as a result, when the wing receives a force acting outward, the wing elastically splays outward.

## Description

### Technical Field

The present invention relates to a lancet assembly constituted from a lancet and a lancet case that houses the former, an injector used in combination with the lancet assembly, and a pricking device constituted from the lancet assembly and the injector. Such device is used in pricking a predetermined portion of a body with a sharp pricking member such as a needle for sampling an amount of body fluid such as blood.

### Background Art

Various pricking devices have been used to take a small amount of blood for the purpose of measuring the blood sugar level of patients with diabetes. Such a device is constituted from a lancet having a pricking member that pricks a predetermined portion of the body of a patient and an injector. The lancet is incorporated in the injector that launches the lancet with the pricking member exposed at the distal end thereof, the lancet being launched toward the predetermined portion by making use of the expanding action of a compressed spring provided in the injector.

When taking an amount of blood by using such a pricking device as described above, particular attention must be paid in the handling of the lancet that has been used. In the lancet that has been used, typically the distal end portion of the pricking member that bears a trace of the patient's blood is exposed from a lancet body. Should a portion of the body of a person other than the patient, for example a nurse who takes the blood sample, accidentally touches the distal end portion of the pricking member, the body portion may be pricked by the distal end portion of the pricking member causing a cut through which the patient's blood may enter the other person's body, thus posing the danger of infection of a disease.

Known pricking devices are not necessarily designed with due consideration given to the handling of the lancet that has been used. For example, it has been proposed to apply a cap on the exposed distal end portion of the pricking member after the pricking operation (refer to Patent Document 1 which will be mentioned later). This device requires the lancet to be handled in the state of its distal end thereof exposed so as to apply the cap thereon, and therefore the danger described above is not eliminated.

Accordingly, the pricking device requires utmost attention in handling the lancet after it has been used, and there is a demand for a pricking device that allows the lancet to be handled after safety thereof has been ensured.
Patent Document 1: U.S. Patent No. 5,385,571

### Disclosure of the Invention

### Problem to Be Solved by the Invention

It is an object of the present invention to provide a pricking device that allows a lancet to be removed from an injector after isolating a protruding distal end portion of a pricking member from its surrounding, rather than removing the lancet from the injector with the distal end portion of the pricking member remaining in the state of protruding from a lancet body, after pricking.

### Means to Solve the Problem

In the first embodiment, the present invention provides a lancet assembly comprising a lancet and a lancet case that houses a portion of the former, wherein
the lancet comprises a lancet body, a lancet cap and a pricking member,
the lancet is a molded article with the pricking member inserted in which the pricking member is disposed in the lancet body and the lancet cap while straddling over these members, and the distal end portion of the pricking member is enclosed by the lancet cap, and the lancet cap and the lancet body are connected together via a weakened portion;
the lancet body comprises a protruding portion,
the lancet case comprises a case body and a pair of wings disposed on both sides of the case body; and
each wing has a stopper that protrudes inward between its front end portion and its rear end portion, the front end portion is connected to the case body and the rear end portion is left free and, as a result, when the stopper receives a force acting outward, the wing is elastically able to splay outward. It is noted that the force acting outward is applied to or in the vicinity of the rear end portion of the wing usually by a wing splaying member as described later when the lancet assembly and an injector according to the present invention. As will be seen clearly from the below detailed description with drawings referred to, the wings extend along the side surfaces of the lancet case or form portions of the side surfaces of the lancet case when no force is applied.

With such lancet assembly according to the present invention, when the lancet is inserted in the lancet case such that the weakened portion is located in the lancet case and the protruding portion of the lancet body is positioned between the stopper of the wing and the rear end opening of the case body,
(a) a portion (a rear portion) of the lancet cap and a portion (particularly a front portion) of the lancet body are housed (therefore, a portion of the lancet is housed) in the lancet case,
(b) when no force is applied to the wing of the lancet case, the protruding portion of the lancet body is able to move back and force between the stopper of the wing and the rear end opening of the lancet case, but due to abutment against the stopper, the protruding portion of the lancet body is unable to move ahead over the stopper, and due to abutment against an inner wall of the rear end opening or abutment against a protruding portion which protrudes inward at the rear end opening, the protruding portion of the lancet body is unable to move rearward over the rear end opening.

It is noted that when the force acting outward is applied to the stopper of the wing, the wing elastically splays outward while the front end portion of the wing is connected to the case body. As a result, the protruding portion of the lancet body does not abut against the stopper as described above, and therefore the protruding portion becomes able to move forward.

The lancet assembly of the present invention surely keeps the state wherein the wings are elastically splayed outward as described above by using an injector into which the lancet assembly is incorporated, and then the pricking operation as to a predetermined portion is carried out by launching a lancet body with a distal end portion of the pricking member exposed so as to move the protruding portion of the lancet body moves forward ahead of the stopper.

After the pricking operation, by removing the force applied to the elastically splayed out wing so as to return the wing toward its original form, the protruding portion of the lancet body again becomes able to move only between the stopper and the rear end opening of the case body by means of the action of the stopper and the protruding portion of the lancet body. By designing the case body such that the exposed distal end portion of the pricking member is located sufficiently away back from the front end opening of the case body, no accidental contact with the distal end portion of the pricking member from the front end opening of the case body is substantially eliminated after the pricking operation with the lancet assembly, the lancet assembly used for the pricking operation can be disposed safely.

It is noted that the removal of the force as described above is carried out automatically when the lancet assembly which was used for the pricking operation is ejected from the injector, so that the risk as to the distal end portion of the pricking member is substantially eliminated with thus ejected lancet assembly.

AS to the lancet assembly according to the present invention, the lancet case described above is preferably constituted by integrally forming the case body and the wings by molding (particularly injection molding) from a resin such as a polypropylene resin, a polyethylene resin, a polystyrene resin, a POM resin (polyacetal resin), a nylon resin, an ABS resin, a polycarbonate resin, a vinyl chloride resin, an elastomer resin, a silicone resin, a rubber based resin, a PBT resin (polybutylene terephthalate resin), a polyester copolymer resin or the like. As for the lancet, the pricking member is usually made of a metal, for example, a stainless steel, and the other portion may be formed of a resin similar to that of the lancet case, and is preferably formed by the injection molding with the pricking member being inserted.

In the lancet assembly according to the present invention, the breakage of the weakened portion of the lancet can be carried out by turning (so-called twisting) the lancet cap and the lancet body along relatively opposite directions around the extending direction of the pricking member. That is, the weakened portion is designated such that the above mentioned turning is sufficient for breaking the weakened portion. For example, a resin material for forming the lancet and/or a thickness of the weakened portion is appropriately selected. After breaking the weakened portion as described above, the distal end portion of the pricking member is exposed.

In order to facilitate the breakage of the weakened portion by turning the lancet cap and the lancet body along the relatively opposite directions, the lancet body and the lancet case are designated such that the lancet body does not turn in the lancet case relatively around the extending direction of the pricking member. Specifically, the lancet body and the lancet cap are adopted such that a portion of an inside wall which defines an inner space of the case body is present inside a trajectory of the lancet body which is formed by rotating the lancet body around the extending direction of the pricking member. In this case, it is preferable that the lancet body is not of an axial symmetry shape with respect to extending direction of the pricking member

In contrast, the lancet body and the lancet cap are preferably designed such that a portion of the lancet cap which is positioned near the lancet cap, namely the rear portion of the lancet cap is able to rotate in the lancet case around the extending direction of the pricking member. Specifically, the lancet body and the lancet cap are such that an inside wall which defines the inner space of the case body is located outside a trajectory of the lancet cap (its rear portion) which is formed by rotating the lancet cap around the extending direction of the pricking member. In this case, it is preferable that the lancet body is of an axial symmetry shape with respect to extending direction of the pricking member (for example, a column form).

As to the inner space of the case body, its cross section perpendicular to the extending direction of the pricking member does preferably not change so much, and it is more preferable that such cross section does substantially not change. For example, the shape of the cross section of the inner space of the case body is made rectangular. In this case, for example, the shape of the cross section of the lancet body has other shape which makes it impossible to rotate in said rectangular shape of the case body (for example other rectangle or a square, a oval or the like) while the rear portion of the lancet cap has a circular cross section which makes it possible to rotate in said rectangular shape.

The lancet comprising the lancet body, the lancet cap and the weakened portion provided therebetween is preferably manufactured by molding a resin with the pricking member being inserted therein (so-called insert molding process) as mentioned previously. This method is advantageous in that it is possible to easily manufacture the lancet in large numbers that comprises the lancet body and the lancet cap that are integrally connected via the weakened portion while the pricking member extends in these members straddling therebetween. The weakened portion is preferably formed by making the thickness smaller in the portion of the resin layer that surrounds the pricking member, for example by forming a notch therein. In other preferable embodiment, the weakened portion can be formed by forming an incision in the resin layer that does not reach the pricking member after forming the resin layer that surrounds the pricking member.

In one preferred embodiment, the lancet cap has a protruding portion that protrudes outward, which protruding portion preferably extends over substantially the entire circumference of the lancet cap. The protruding portion has an external profile larger than an inner profile of the front end opening of the lancet case, so that the protruding portion cannot enter the inner space of the lancet case through the front end opening since the protruding portion abuts against the front end of the lancet case so that no further movement of the lancet rearward relative to the lancet case is prevented when the lancet is inserted in the lancet case so as to form the lancet assembly.

In one preferred embodiment, the stopper of the wing has a tapered shape which extends inward with respect to the rearward direction of the lancet case. In this embodiment, when the lancet body is inserted in lancet case through its front end opening and moved rearward relative to the lancet case so as to make the protruding portion of the lancet body abut against the stopper and then lancet body is intentionally moved further back by applying a rearward force to the lancet, the protruding portion presses a surface of the tapered shape outward, so that the wing elastically splays out, and then returns to its original shape after it has passed over the tapered shape.

In one preferred embodiment, the lancet case comprises a front end opening and a rear end opening stopper of the wing, and when the lancet body is inserted in the lancet case through the front end opening and moved therein rearward, the protruding portion of the lancet body abuts against a wall which defines the rear end opening of the lancet case. In this embodiment, the profile of the protruding portion of the lancet body is larger than an inner profile of the rear end opening of the lancet case. As a result, a further rearward movement of the lancet body in the lancet case becomes substantially impossible. That is, the lancet does not get out of the rear end opening of the lancet case.

In one preferred embodiment, the lancet case comprises a protrusion behind the front end portion. The protrusion fits in a recess which is provided inside and behind an front end opening of the injector, so that lancet assembly is loaded in the injector. In other embodiment, the lancet case comprises a recess and the injector comprises a protrusion behind the front end opening of the injector, which protrusion fits in the recess of the lancet case. In other words, one of two members which get in the fitting or engaging relationship with each other is present behind the front end portion while the other member is located inside and behind the front end opening of the injector. With such engaging or fitting members, the lancet is mounted on the injector in place by fitting in fitting in with each other, for example by snap fitting when the lancet assembly is inserted in the injector.

In the present invention, the lancet case is preferably formed by molding of a resin , particularly injection molding of a resin. When such molding is used, the lancet case is readily prepared wherein the front end portion of the wing is connected to the case body together, and also the nature of the wing to elastically spay out is readily ensured.

In the present invention, the lancet body of the lancet comprises a rear end portion which fits in a front end portion of a plunger of the injector according to the present invention which will be explained below. The plunger launches the lancet. Such rear end portion comprises a protrusion or a recess around its outside, and such protrusion or the recess fits in a recess or a protrusion which is provided on the front end portion of the plunger complementarily to the former protrusion or the recess. As to the relationship of such protrusion and the recess, the above mentioned relationship between said "two members which get in the fitting or engaging relationship with each other" is similarly applicable.

With such members, namely the protrusion and the recess, when the lancet assembly is inserted in the injector through its front end opening, and the rear end portion of the lancet body is moved rearward so as to abut against the front end portion of the plunger, and further moved rearward, the rear end portion of the lancet body is such that it fits in the front end portion of the plunger.

In the second aspect, the present invention provides the injector which is used in combination with the lancet assembly described above and also below so as to launch the lancet body with a distal end portion of the pricking member exposed,
which injector comprises a member which splays (or expands) outward each wing (corresponding to the wing splaying member described above) therein, and
when the lancet assembly is inserted rearward in the injector through its front end portion, each wing of the lancet case is such that it elastically splays out by means of said member which splays outward the wing.

The member which splays out each wing elastically splays out or expands the wing while the front end portion of the wing is in the condition in which it is connected to the case body, so that the wing extends rearward obliquely from the case body.

In one embodiment, the wing splaying member is a pair of wedge-shaped member which are located within the injector,
each wedge-shaped member has a tapered off shape (toward its front) while separated from the other wedge-shaped member so that it defines a slope which is inclined (inward) toward the front of the injector, and
when the lancet assembly is inserted rearward in the injector through its front end opening, the rear end portion of each wing of the lancet case is located so as to mount on the front end portion of the slope of the wedge-shaped member, and then when such insertion is further continued, the rear end portion of the wing slides on the slope, so that the wing elastically splays outward. The provision of such wedge-shaped members as described above makes it possible to automatically splay out the wings only by inserting the lancet assembly, which is convenient.

In the state wherein the wings are in the splaying out condition, the forward movement of the lancet body cannot be prevented by the abutment of the protruding portions of the lancet body against the stoppers of the wings when the lancet body moves along the pricking direction.

In one embodiment, it is preferable that the injector further comprises an ejector that is capable of applying a force acting forward on the lancet case that has been inserted. By moving such an ejector forward, a force can be applied to the rear end portion of the lancet case in the state where for example a bump disposed behind the front portion of the lancet case is fitting in for example an recess at the front end opening of the injector.

When the force is applied to the rear end portion of the lancet case, the bump as fitting in the recess comes out of the recess, so that the lancet case is moved forward. As a result, the force which has been applied to the wings by the members which splay out the wing is removed, so that the wings return to their original forms. Then, the ejector is further moved forward, so that the lancet assembly loaded in the injector is dischargede from the injector.

In one embodiment, the member which is able to apply a forward force to the loaded lancet case abuts against the rear end portion of the lancet case when the loading of the lancet assembly in the injector has been completed or when the ejector is moved forward.

Particularly, it is preferable that a member which is able to apply a forward force to the rear end portion of the inserted lancet case, preferably a rear end portion of a side of the lancet case on which side no wing is present. For example, the ejector abuts against the rear end portion of a side surface of the lancet case which surface is adjacent to the side surface on which the wing is present.

In one embodiment, the plunger comprises a spring between a protrusion provided around an intermediate portion of the plunger and a rear partition provided in the injector housing,
upon loading of the lancet assembly into the injector, the rear end of the lancet abuts against the front end of the plunger, which is moved rearward so that the spring is compressed by such plunger, and the protrusion of the plunger moves a rear end of a trigger lever (or a shoulder at a rear portion of the trigger lever in the embodiment in the drawing which is referred to below) outward to which a inward force is applied, and then when the protrusion is further moved rearward beyond the rear end (or the shoulder) of the trigger lever, the rear end of the trigger lever is returned inward, so that the protrusion (in the form of for example a flange) is engaged with the rear end of the trigger lever while the spring is kept in the compressed condition,
after such engagement, a rearward force is applied to the rear end of the lancet body due to the plunger being impossible to move back, so that the rear end portion of the lancet body is grasped by the plunger. In other embodiment, the above described engagement may be caused after the rear end portion of the lancet body is grasped by the plunger.

After such engagement is achieved, when the engagement is removed by moving the rear end (or the shoulder) of the trigger lever outward, the spring in the compressed condition expands at once. Such expansion is used for launching the lancet body which is in the free condition as described above while the distal end portion of the pricking member is exposed so as to prick a predetermined portion.

In one embodiment, the injector according to the present invention comprises
a pricking depth adjusting drum disposed behind and adjacent to the rear partition in the injector housing,
the rear end portion of the plunger extends through the drum,
the drum has a ring-shaped member on the inside at the front end of the drum which member is capable of rotating around the plunger,
the length of the ring-shaped member in the pricking direction changes along the circumferential direction thereof continuously or stepwise,
the rear end of plunger has a hitting member that strikes the rear end surface in a portion along the circumferential direction of the ring-shaped member,
when the plunger moves forward for pricking, the hitting member on the rear end of plunger strikes a portion of the rear end surface of the ring-shaped member in the drum, and is therefore unable to move further forward,
a portion of the rear end surface of the ring-shaped member as an object which the hitting member provided on the rear end of the plunger strikes can be changed by causing the ring-shaped member to rotate around the plunger by means of the drum rotation, so that the length between the rear partition of the housing of the injector and the rear end of the plunger is changed upon striking of the hitting member. Therefore, when the hitting member on the rear end of the plunger strikes a portion of the rear end surface of the ring-shaped member in the drum, the length of a plunger portion which is located ahead of the rear partition is changed, that is the pricking depth is changed.

Since the length of the plunger itself (the entire length) is fixed, the length of the distal end portion of the pricking member which protrudes out of the front end opening of the lancet case can be changed upon pricking by being able to change the distance from the rear partition up to the rear end of the plunger.

It is noted that the present invention provides the lancet that constitutes the lancet assembly of the present invention described above, the lancet case that constitutes the lancet assembly of the present invention described above, the kit of the lancet and the lancet case for constituting the lancet assembly of the present invention described above, the pricking device composed of the lancet assembly of the present invention described above and the injector of the present invention described above, and the kit of the pricking device constituted from the lancet assembly of the present invention described above and the injector of the present invention described above.

### Effects of the Invention

In the case wherein the lancet assembly of the present invention and the injector of the present invention are combined and used as the pricking device of the present invention, preparation for the pricking operation is completed only by merely inserting the lancet assembly so as to load it into the injector and twisting off the lancet cap. The pricking device also allows it, after the pricking operation, to remove the distal end portion of the pricking member from the injector in a state of being substantially isolated in the lancet case from the surrounding, although it is still protruding from the lancet body. It is noted that "the state of being substantially isolated from the surrounding" means that the distal end portion of the pricking member is located at a position sufficiently away from the front end opening of the lancet case, and therefore a person who takes the blood sample does not normally touch the distal end portion in the routine pricking operations.

### Brief Description of Drawings

Fig. 1 is a schematic perspective view of a lancet assembly of the present invention.
Fig. 2 is a schematic perspective view of a lancet assembly of the present invention, with a near side half of the lancet case being cut away (wings are closed) for the ease of understanding the structure.
Fig. 3 is a schematic perspective view of a lancet assembly of the present invention with its near side half being cut away.
Fig. 4 is a schematic perspective view of an injector into which the lancet assembly is loaded.
Fig. 5 is a schematic perspective view of the injector with the lancet assembly being loaded therein.
Fig. 6 is a schematic perspective view of a lancet that constitutes the lancet assembly of the present invention.
Fig. 7 is a schematic perspective view of the state of the lancet assembly being inserted with a near side housing half of the injector housing being removed.
Fig. 8 is a schematic perspective view of the state of the lancet assembly being inserted (the same state as in Fig. 7) with a near side half of the lancet case being cut away.
Fig. 9 is a schematic perspective view of the state in which the lancet assembly has just been loaded in the injector similarly to Fig. 7.
Fig. 10 is a schematic perspective view of the state in which the lancet assembly has just been loaded in the injector similarly to Fig. 8.
Fig. 11 is a schematic perspective view of the state in which the lancet cap has been removed from the state shown in Fig. 9, similarly to Fig. 7.
Fig. 12 is a schematic perspective view of the state in which the lancet cap has been removed from the state shown in Fig. 10, similarly to Fig. 8.
Fig. 13 is a schematic perspective view of the state in which the distal end portion of the pricking member has just protruded from the front end opening of the lancet case, similarly to Fig. 7.
Fig. 14 is a schematic perspective view of the state in which the distal end portion of the pricking member has just protruded from the front end opening of the lancet case, similarly to Fig. 8.
Fig. 15 is a schematic perspective view of the state in which the lancet body is removed rearward from the state shown in Fig. 13, similarly to Fig. 7.
Fig. 16 is a schematic perspective view of the state in which the lancet body is removed rearward from the state shown in Fig. 14, similarly to Fig. 8.
Fig. 17 is a schematic perspective view of the state in which the lancet case is being moved forward by means of an ejector, similarly to Fig.7.
Fig. 18 is a schematic perspective view of the state in which the lancet case is being moved forward by means of an ejector, similarly to Fig.8.
Fig. 19 is a schematic perspective view of the plunger.
Fig. 20 is a schematic perspective view of the state of a housing half that constitutes the injector housing.
Fig. 21 is a schematic perspective view showing the lancet assembly in the state where the wings are splayed out.
Fig. 22 is a schematic perspective view of the ejector.
Fig. 23 is a schematic perspective view of the lancet assembly which has been discharged from the injector.
Fig. 24 is a schematic perspective view of a ring-shaped member and a hitting member.

### [Description of Reference Numerals]

- 100:: Lancet assembly
- 102:: Lancet case
- 104:: Front end opening
- 106:: Front end opening
- 107:: Rear end opening
- 108:: Rear end portion
- 110:: Front end portion
- 112:: Bump
- 114:: Case body
- 116:: Wing
- 118:: Front end portion
- 120:: Rear end portion
- 122:: Stopper
- 124:: Sloped surface
- 126:: Rear portion surface
- 128:: Side surface
- 130:: Rear end surface
- 132:: Rear end wall
- 140:: Rail
- 200:: Lancet
- 202:: Rear end portion
- 204:: Lancet body
- 206:: Lancet cap
- 208:: Weakened portion
- 210:: Pricking member
- 212:: Protruding portion
- 218:: Bump
- 220:: Bump
- 222:: Protruding portion
- 300:: Injector
- 301:: Plate-like portion
- 302:: Front end opening
- 303:: Protrusion
- 304:: Protruding portion
- 306,: 308: Injector housing half
- 309:: Housing
- 310:: Plunger
- 314:: Front end portion
- 315:: Seat surface
- 316:: Recess
- 320,: 322: Leg
- 324:: Protruding portion
- 330:: Shoulder
- 340:: Wedge-shaped member
- 341:: Front end portion
- 342:: Slope
- 343:: Cut away defining wall
- 344:: Front partition
- 346:: Protrusion
- 348:: Recess
- 350:: Push button
- 351:: Base plate
- 354:: Operation button
- 356:: Pusher
- 358:: Rear partition
- 360:: Pricking depth adjusting drum
- 361:: Ring-shaped member
- 362:: Rear end portion of plunger
- 363:: Opening
- 365:: Hitting member
- 367, 367':: Hitting portion
- 369:: rotation limiting member
- 371, 371', 373, 373':: Step

### Best Modes for Carrying Out the Invention

The lancet assembly of the present invention, the lancet and the lancet case that constitute the same, the injector that is used in combination with the lancet assembly, and a pricking device of the present invention will be described below in detail with reference to the accompanying drawings. The moving direction of the lancet for pricking (that is, the direction of the pricking member which is moving for pricking, which is also referred to pricking direction) corresponds to a "front" meaning direction, and the opposite direction corresponds to a "rear" meaning direction, and the terms relating to the directions in the present specification are used based on such directions.

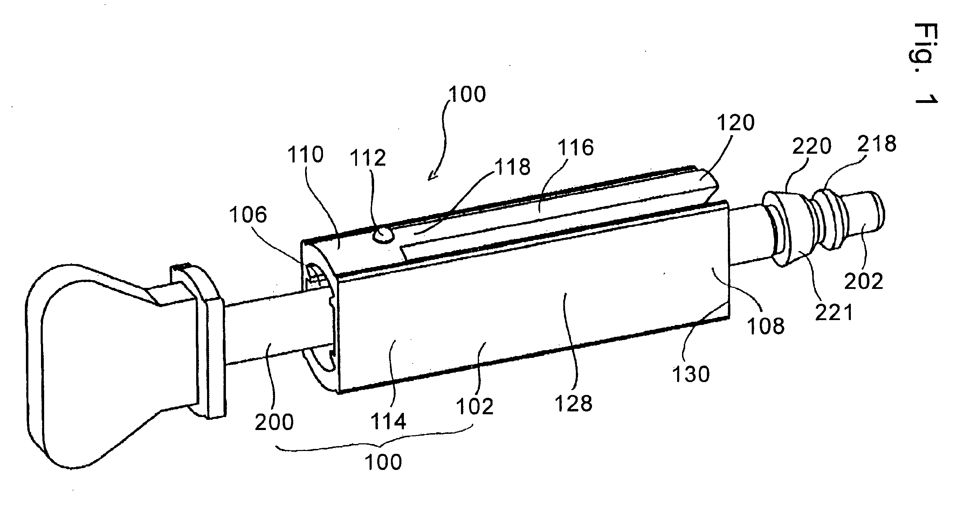

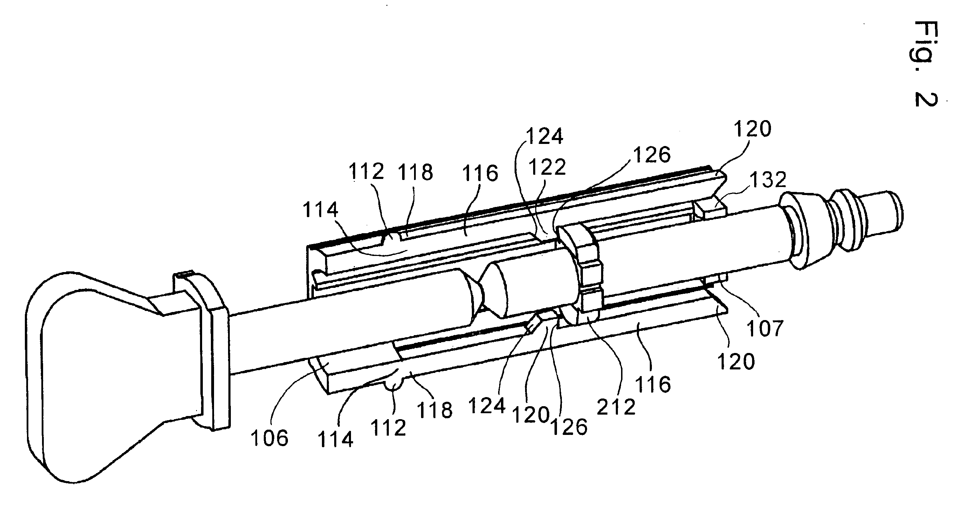

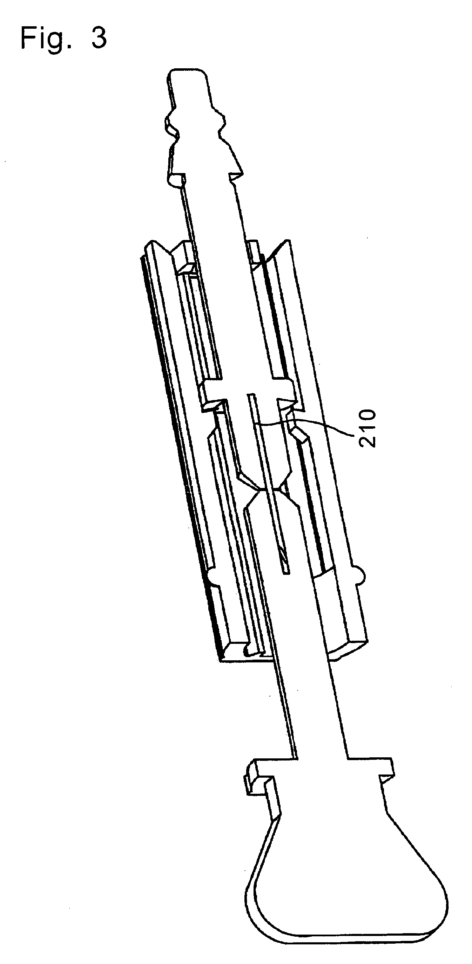

The lancet assembly according to the present invention is schematically shown in Fig. 1 in a perspective view. It is noted that for the purpose of readily understanding the structure of the lancet assembly, Fig. 2 shows the lancet assembly with a near side half of the lancet case being cut away in a schematic perspective view while Fig.3 shows the lancet assembly with a near side half thereof being cut away in a schematic perspective view. Those drawings show the lancet assembly in a completed form as an assembly wherein the lancet 200 is incorporated in the lancet case 102. The lancet case 102 is in the form of for example a rectangular column as a whole, and the rear end portion 202 of the lancet 200 is inserted rearward in the inside of the lancet case through the front end opening 106 of the lancet case, and thereby the lancet assembly 100 is assembled. The lancet case 102 has an opening 106 (at the front end) and an opening 107 (at the rear end), and a predetermined portion (for example, a finger tip) is applied onto the front end opening 106 upon pricking.

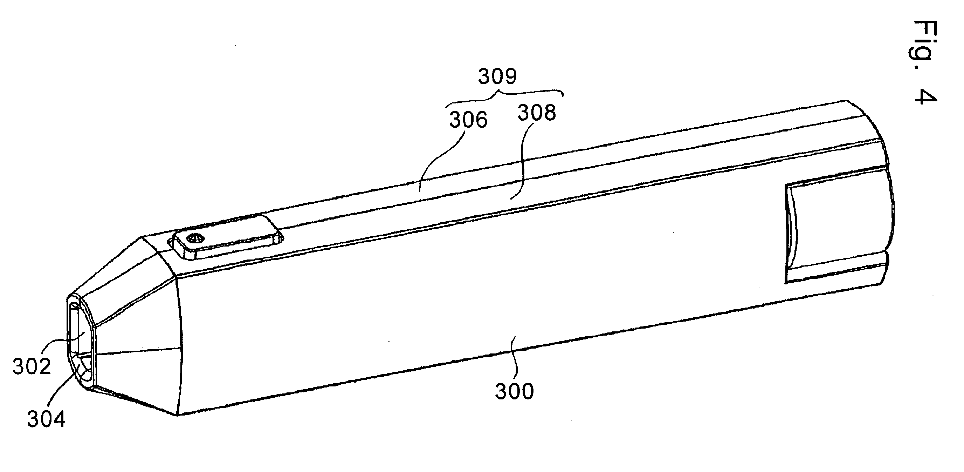

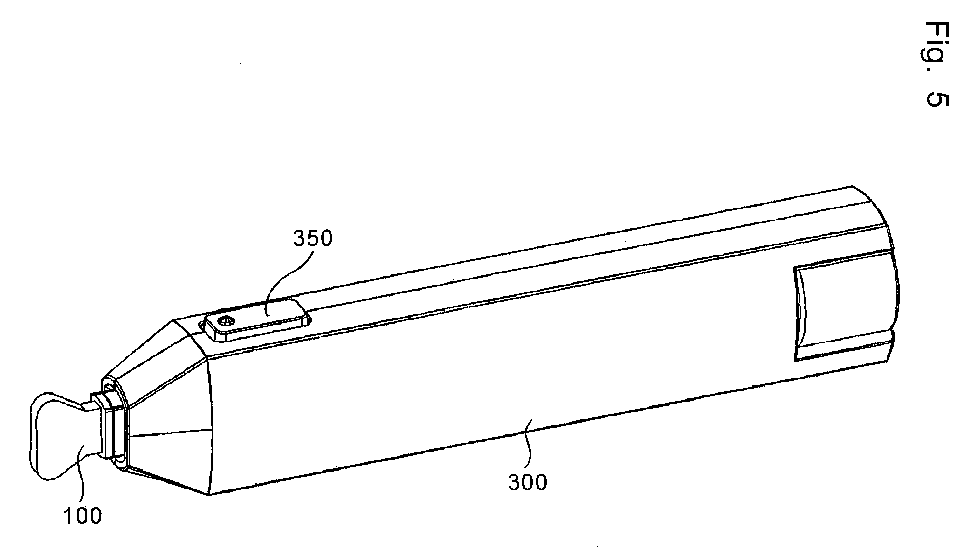

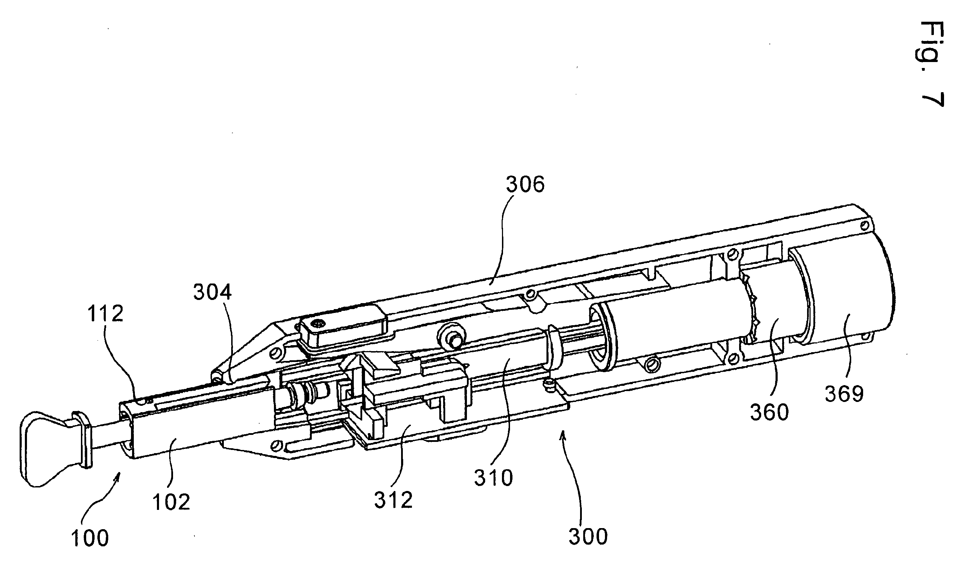

Fig. 4 schematically shows the injector 300 into which the lancet assembly is loaded in a perspective view, and Fig. 5 schematically shows the injector 300 with the lancet assembly being loaded in a perspective view. As described below, the injector 300 includes a mechanism which launches the lancet in a space of the housing 309 defined by injector housing halves 306 and 308. Loading of the lancet assembly 100 in the injector 300 is carried out by inserting, through the front end opening 302 of the lancet case toward the inside thereof, the rear end portion 108 of the lancet case first, and then the most portion of the lancet. The lancet case 102 has a bump (or protrusion) 112 which is positioned behind the front end portion 110 of the lancet case, and such bump 112 gets over the protruding portion (304, see Fig. 7) positioned on the injector inside behind the front end opening 302 of the injector, preferably followed by fitting in a complementary recess positioned behind the protrusion, so that the loading of the lancet assembly into the injector is completed to be in the state as shown in Fig. 5.

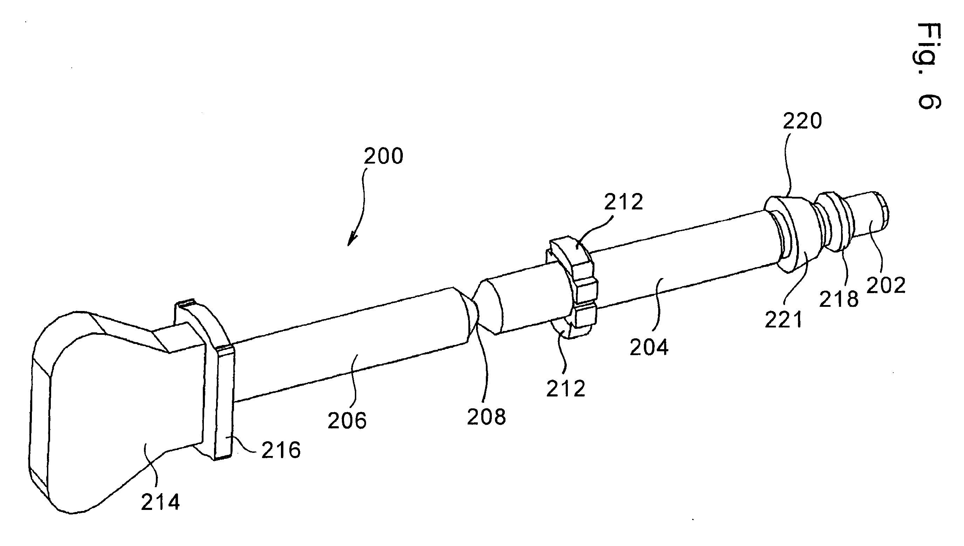

The lancet 200 shown in Fig. 6 in a perspective view which forms the lancet assembly according to the present invention comprises a lancet body and lancet cap 206, which are connected together through a weakened portion 208. In the lancet 200, the pricking member 210 (see Fig. 3) is disposed to extend in the lancet body 204 and the lancet cap 206 while straddling over these members, and the distal end portion of the pricking member 210 is enclosed by the lancet cap 206. Such lancet 200 is formed by molding a resin (a resin molded product) with the pricking member 210 inserted therein. The lancet body 204 has a protruding portions 212 on the outside thereof.

The rear end portion 202 of the lancet body 204 is configured such that it fits in the front end portion 314 of the plunger 310 in the injector 300. The rear end portion 202 comprises a bump 218 around its outside, and the bump is configured such that it fits in a complementary recess 316. The relationship between the bump and the recess as described is similar to that of the above mentioned "two members which get in the fitting or engaging relationship with each other", and thus what is applicable to the latter relationship is similarly applicable to the former relationship. It is possible to use a recess in place of the bump, and to use a bump in place of the recess.

The lancet body further comprises other bump 220 in front of the bump 218. A rear surface 221 of this bump 220 is in the condition of abutment against a seat surface 215 which is provide at the front end of the plunger 310 when the loading of the lancet assembly is completed.

With the lancet as described, the weakened portion 208 is configured such that it is broken by applying a force which turns the lancet body 204 and the lancet cap 206 in opposite directions around the extending direction of the pricking member 210, or in addition or in place of such force, by applying a force to make these members separate from each other along the pricking direction of the pricking member. After breaking the weakened portion, when the lancet cap 206 is brought away from the lancet body 204, the distal end portion of the pricking member 210 is exposed from the lancet body 204.

The lancet case 102 which forms the lancet assembly according to the present invention comprises a pair of the wings 116 which are located on the side surfaces, preferably the opposing side surface of the case body 114. Such wings preferably extend along the side surfaces of the lancet case (thus, the wings substantially form portions of the side surfaces of the lancet case). Each wing 116 comprises the stopper 122 which protrudes inward between its front end portion 118 and its rear end portion 120. The front end portion 118 is connected to the case body 114, and the rear end portion 120 is free. As a result, when the lancet 200 is inserted in the lancet case 102 through its front end opening 106, the protruding portions 212 of the lancet body 204 abut against the stoppers 122, and the lancet is moved further rearward, the protruding portions 212 go up over slopes 124 of the stoppers 122, so that outward forces are applied to the wings, and thereby the wings 116 are elastically splayed outward.

Thereafter, when the protruding portions 212 pass over the stoppers 122, the wings 116 return to their original forms to be in the state as shown in Fig. 2. The rear surface 126 of the stopper 122 is at right angle relative to the wing 116 or at an angle close to such angle. As a result, in the state as shown in Fig. 2, even when a forward force is applied to the lancet, the protruding portion 212 abuts against the rear surface 126 so that the lancet is not able to move forward any further, and therefore the forward movement of the lancet is prevented. Once the protruding portion 212 has passed over the stopper 120 to be present behind the stopper, the protruding portion cannot move ahead of the stopper 120. In the state wherein the protruding portion 212 has just passed over the stopper 120, the rear end portions with the two bumps (or protrusions) 218 and 220 in the condition of extending rearward (namely outward) from the rear end opening 107 of the lancet case 102.

The lancet cap 206 has a grip portion on its front end portion 214 which is pinched when the weakened portion 208 is broken as described above, and the lancet cap has a protruding portion 216 behind such grip portion. The protruding portion 216 is preferably in the form of a flange as shown, and it abuts against a portion which defines the front end opening 106 of the lancet case 102, so that the lancet 200 cannot move rearward any further relative to the lancet case 102.

Thus, when a forward force is applied to the lancet 200 in the state of the lancet assembly 100 as assembled as shown in Fig. 2, the lancet does not get out of the front end opening 106 of the lancet case 102. Also, the lancet does not get out of the rear end opening 107 even when a rearward force is applied to the lancet 200.

The lancet assembly which has been assembled as described above is inserted in the injector 300 through its front end opening 302 as shown in Fig. 4, so that the state shown in Fig. 5 is achieved after the bumps 112 of the lancet passed over the protruding portions 304, and this states corresponds to the completion of loading.

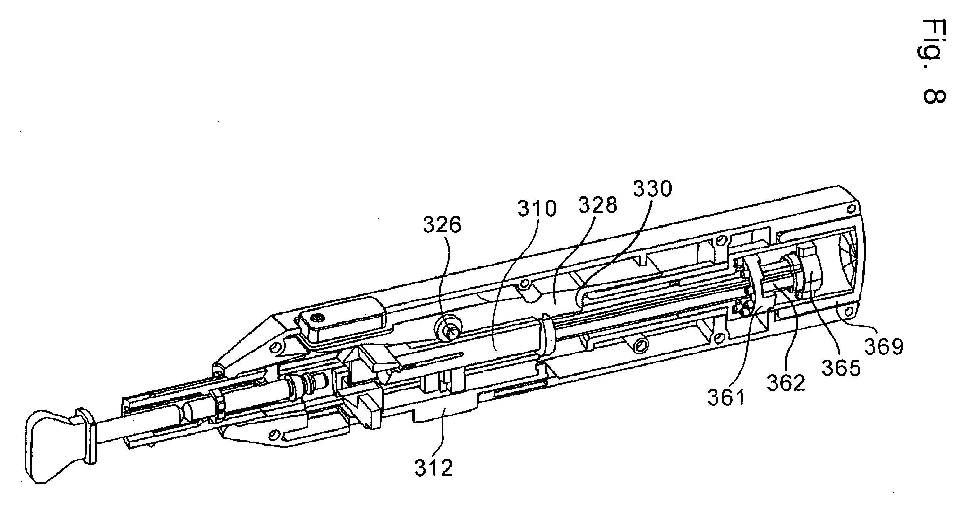

Figs. 7 and 8 show, in perspective views, steps on the way to loading of the lancet assembly in the injector. For the ease of understanding the relationship between the lancet assembly 100 and the injector 300 as well as the inner structure of the injector, Fig. 7 shows the state where the housing half located on the near side of the injector housing is removed. For the ease of understanding the pricking depth adjusting mechanism which will be described later, Fig. 8 shows the state where the near side half of the lancet case 102 is cut away, and for the ease of understanding the state of the plunger 310 which launches the lancet, a near side half of the ejector 312 described below is cut away and also a near side half of a drum rotation limiter 380 which will be described later is removed.

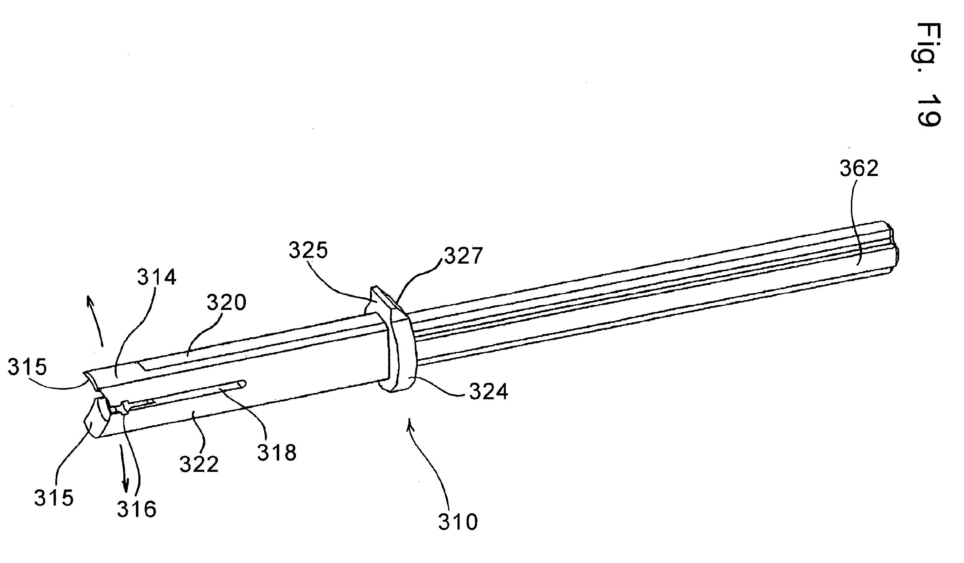

As shown in Fig. 19, the front end portion 314 of the plunger 310 is bifurcated into two portions, and a first leg 320 and a second leg 322 that define a small gap 318 therebetween. The plunger is formed by, for example, molding a plastic material, so that the first leg 320 and the second leg 322 are capable of elastically deforming. When these legs are subjected to forces acting outwardly as indicated by the arrows, these legs deflect outward so as to expand the gap 318.

With this configuration, when the lancet assembly 100 shown in Fig. 1 is inserted into the injector 300 as shown in Fig. 7, the rear end portion 202 of the lancet body 204 approaches, after the state of Fig. 7, the seat surfaces 315 of the front end portion 314 of the plunger in the state as shown in Fig. 19, and then abuts against the surfaces 315. An attempt of inserting the lancet assembly thereafter causes the rear end portion 202 of the lancet body 204 to apply a force of pressing backward to the seat surfaces 315. Since the seat surfaces 315 are inclined, this force also generates a force that acts in the direction indicated by the arrows, so that the leg 320 and the leg 322 elastically splay toward the outside.

When the first leg 320 and the second leg 322 splay out as described above, the rear end portion 202 of the lancet body 204 can move further rearward, so that the bump 218 of the rear end portion 202 of the lancet body 204 fits into the recesses 316 and, at the same time, the legs that have expanded outward move to return somewhat so as to restore the original shape. As a result, the rear end portion 202 is held between the legs 320 and 322 and snugly fits into the gap 318. At this time, the surface 221 that opposes to the front side of the protruding portion 218 of the lancet body makes close contact with the seat surfaces 315 provided on the distal end of the plunger 310.

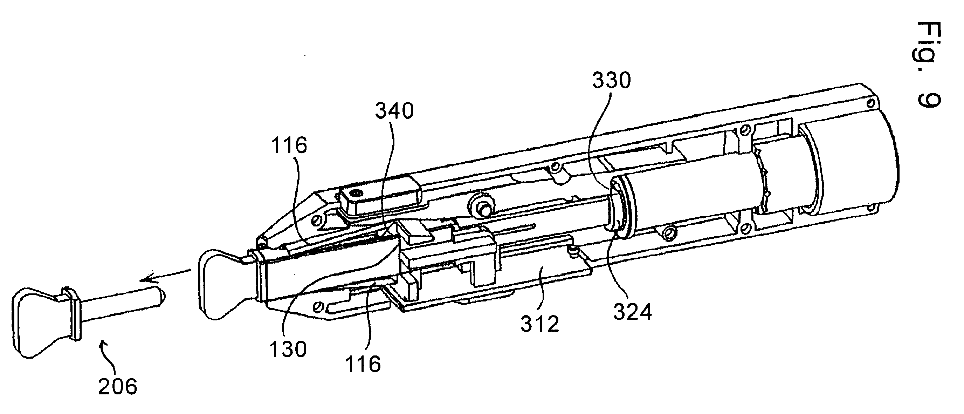

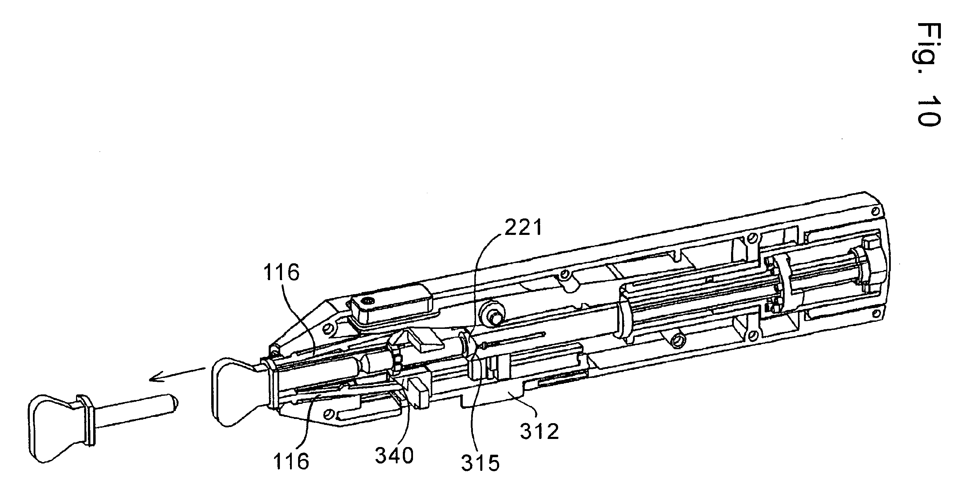

Figs. 9 and 10 show the state in which the lancet assembly 100 has just been loaded in the injector 300, similarly to Figs. 7 and 8. As seen from Fig. 10, the rear inclined surface 221 of the bump 220 of the lancet body 204 is abutting against the seat surfaces 315 of the front end of the plunger 310.

The plunger 310 has a launching spring (not shown) disposed around the plunger 310 behind the protruding portion 324 provided around an intermediate portion of the plunger (for example between protruding portion 324 and the rear partition 358). When the lancet assembly is loaded in the injector, the rear end portion 202 of the lancet which abuts against the front end portion 314 of the plunger moves the plunger back so as to compress the spring. Upon such compression, the protruding portion 324 of the plunger is moved backward on the lower edge of the trigger lever 328, while exerting a force that pushes upward the trigger lever pivoted by a shaft 326 against the force exerted downward by the trigger lever 328.

Then immediately after the protrusion 324 has passed below the shoulder 330 located at the rear end of the trigger lever, the shoulder 330 of the trigger lever abruptly moves downward (by means of the above mentioned downward force), so that the protruding portion is in the condition of being engaged with the shoulder 330. In this situation, in spite of the fact that the forward force is applied to the plunger by the compressed launching spring, the state as shown in Fig. 9 in which the protruding portion 324 is engaged with the shoulder 330 is kept even though the force which is applied to move the plunger rearward is removed. That is, the so-called cocking state is achieved for launching of the lancet.

In the state wherein the protruding portion 324 is engaged with the shoulder 330 located on the rear end of the trigger lever, it is preferable that the gap (or space) 318 at the front of the plunger does not expand, and therefore it is preferable that the first leg 320 and the second leg 322 of the plunger do not deform so as to splay outward. In order to prevent such expansion, it is preferable that the front partition 344 provided in the housing has a cut away portion 345, and a wall 343 which defines the cut way portion just surrounds the front end portion 314 of the plunger 310, so that the front end portion 314 of the plunger does not expand.

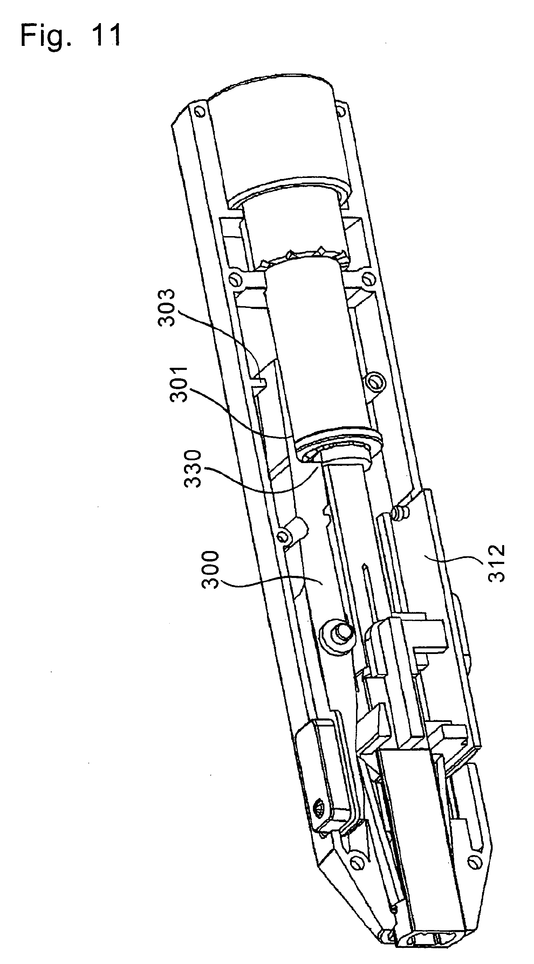

It is noted that in the embodiment illustrated, a plate-shaped member 301 extends toward the rear from the upper edge of the end surface of the shoulder 330 located at rear end of the trigger lever. The plate-shaped member 301 is adapted such that when a button 350 is depressed inward to launch the lancet, the plate-shaped member 301 presses the protrusion 303 in the housing and undergoes elastic deformation, so that a force is exerted by the deformation to push back the button (see Fig. 11).

When the rear end portion of the lancet body is moved rearward so as to make it abut against the front end portion of the plunger, and then the lancet body is moved further rearward, the rear end portion of the lancet is such that it fits in the front end portion of the plunger. It is noted that such fitting of the rear end portion of the lancet body may occur after the rear end portion of the lancet body move the plunger rearward so as to make no further rearward movement impossible. In other embodiment, the fitting may occur first, and then the rearward movement of the lancet body moves the plunger rearward.

At the completion of the loading of the lancet assembly 100 into the injector 300 as shown in Figs.9 and 10, the attention is to be paid to that the wings 116 on the both sides of the lancet case 102 are in the condition of being splayed out. The reason why the wings are in the splayed out condition is that a pair of wedge-shaped members 340 are provided as the wing splay member in the injector. Such wedge-shaped members are separated from each other and have a tapered off form (that is, sharpend form with respect to the front direction), so that they form sloped surfaces 342 which are inclined with respect to the front of the injector.

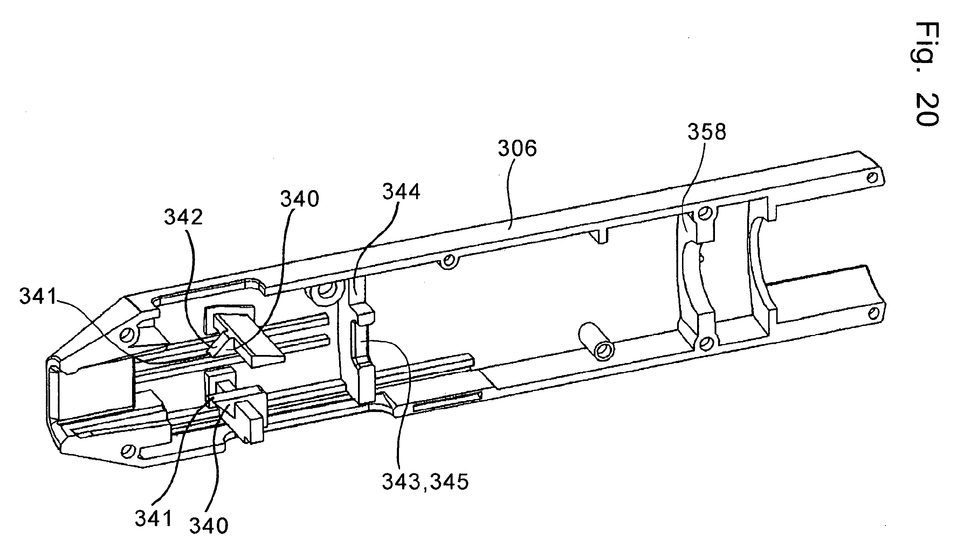

The wedge-shaped members are provided in the housing half 306 which is schematically shown in Fig. 20. In the shown embodiment, the wedge-shaped members 340 are provided by fitting a member as a separate member in the housing half 306. The wedge-shaped member is configured such that the front ends 341 of the wedge-shaped members 340 enter inside the free rear ends 120 of the wings 116 of the lancet case 102 immediately before the loading of the lancet assembly 100 into the injector 300. Thereafter, when the lancet assembly is moved further backward, the rear ends of the wings 116 slide along and on the sloped surfaces 342 of the wedge-shaped members 340, so that the wings 116 expand outward. Thus it is advantageous to provide the wedge-shaped members since it makes possible to automatically expand the wings 116 simply by inserting the lancet assembly rearward for setting.

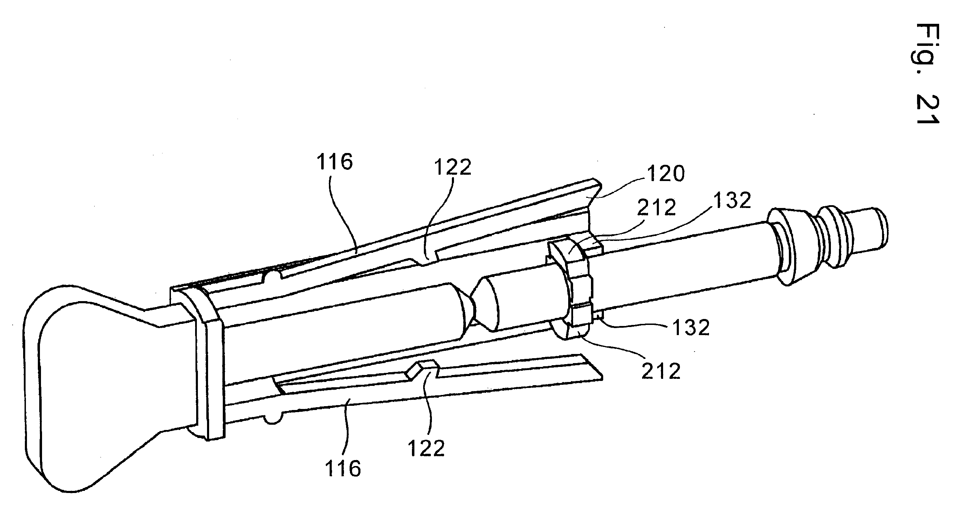

Fig. 21 shows in a schematic perspective view the state of the lancet assembly 100 where the wings 116 are in the splayed out condition. It is noted that the lancet case 102 is shown with its near side half of the lancet case 102 is cut away for understanding the state of the lancet located inside the lancet case. As easily seen, the positions of the stoppers 122 are shifted outward because of splaying out of the wings 116 when compared with the state in which the wings are not splayed out (as shown for example in Fig. 2). As a result, the stoppers 122 cannot function as the stoppers any more. Therefore, in the state shown in Fig. 21, when the lancet is to move forward, the the protruding portions 212 of the lancet body 204 do not hit the stoppers 122, so that the forward movement of the lancet is not prevented.

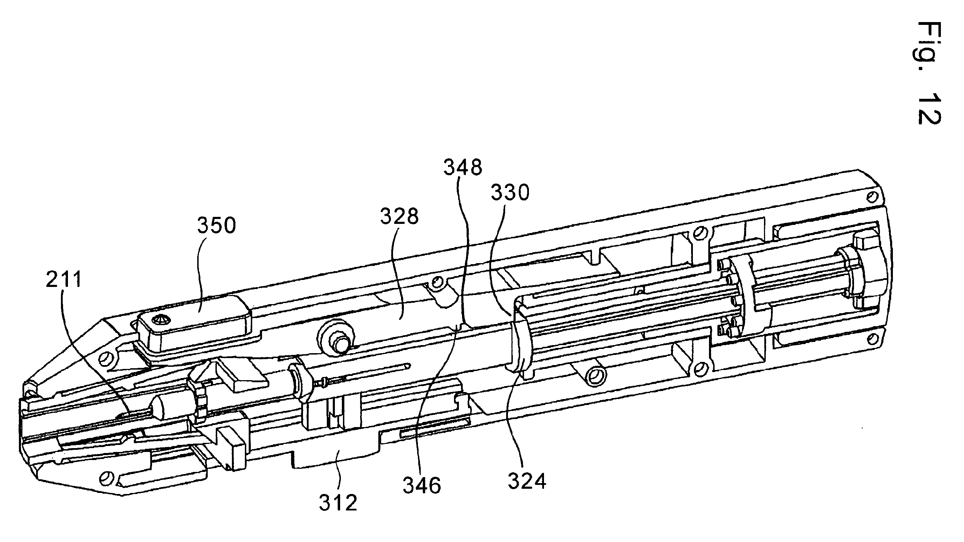

After the loading of the lancet assembly 100 into the injector is completed described above, the lancet cap is removed. The weakened portion is broken by applying forces to the lancet body 204 and the lancet cap 206 in opposite directions around the extending direction of the pricking member, or pulling them so that these members are separated away from each other along extending direction of the pricking member. Then, when the lancet cap 206 is removed away from the lancet body 204, the distal end portion of the pricking member 210 is exposed from the lancet body 204 (see Fig. 12). In this way, the lancet cap performs a function of covering the distal end portion of the pricking member in advance.

In order to make it easy to twist off the lancet cap 206 by applying the force in the opposite directions around extending direction of the pricking member in the lancet case 102, it is preferable that the lancet body 204 is formed with its cross section of which shape makes it impossible to rotate in the lancet case, while the rear portion of the lancet cap 206 located in the lancet case has a cross section of a shape that enables it to rotate in the lancet case. For example, in the case where the lancet case that accommodates the lancet body and the lancet cap has a rectangular cross section, the lancet body has a rectangular cross section that makes it impossible to rotate therein and the rear portion of the lancet cap has a circular cross section.

Figs. 11 and 12 schematically show the state wherein the lancet cap is removed from the state shown in Figs. 9 and 10, similarly to Figs. 7 and 8. As seen from Fig. 12, the distal end portion 211 of the pricking member 210 is exposed in the lancet case, As seen from the comparison of Fig. 11 with Fig. 12, the exposed tip of the distal end portion of the pricking member is located sufficiently back away from the front end opening 106 of the lancet case 102.

As described above, the launching spring is kept in the compressed condition by the engagement of the protruding portion 324 of the plunger with the shoulder 330 of the trigger lever. Accordingly, in the state shown in Figs. 11 and 12, the predetermined portion (for example, a finger tip) to be pricked is applied onto the front end opening 106 of the case body 114, and then when the engagement of the trigger lever 328 is removed, the launching spring that has been compressed expands instantly. As a result, the plunger 310 moves forward so that the lancet body 204 with the distal end portion of the pricking member being exposed moves instantly forward and protrudes out of the front end opening 106 of the lancet case 102, thereby pricking the predetermined portion applied thereto.

It is noted that the engaged state can be canceled by depressing the push button 350 downward, which is provided at the front end of the trigger lever. The trigger lever can rotate around a rotary shaft 326, and may have a compressed spring under the push button 350. In this case, when no force is applied to the button from its outside, an upward (thus, outward) force is applied to a portion of the trigger lever in front of the rotary shaft 326 as well as the bush button, while a downward (thus, inward) force is applied to a portion of the trigger lever behind the shaft. It is noted that by the similar action may be carried out by using the plate-like member 301 as described above, but reliability of such action is improved by using such spring.

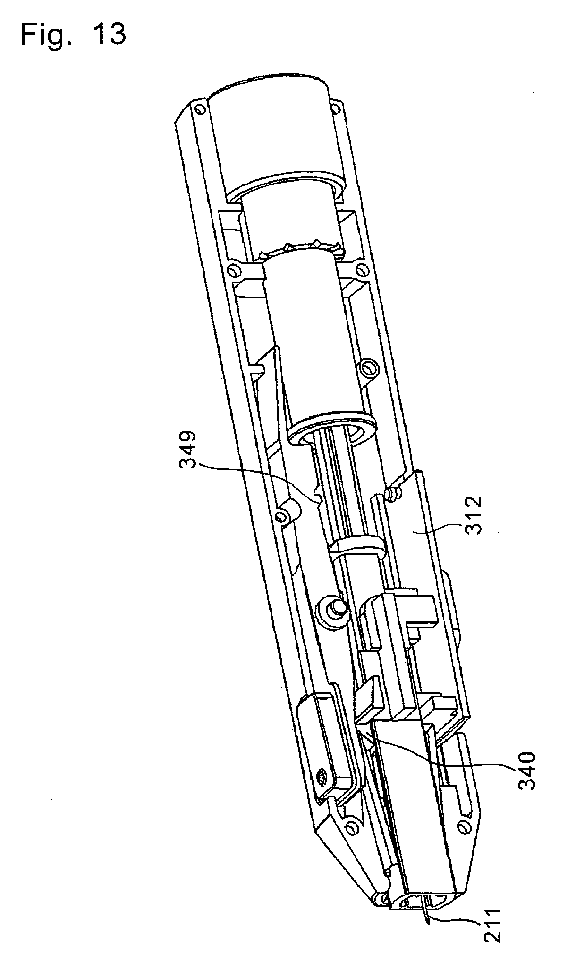

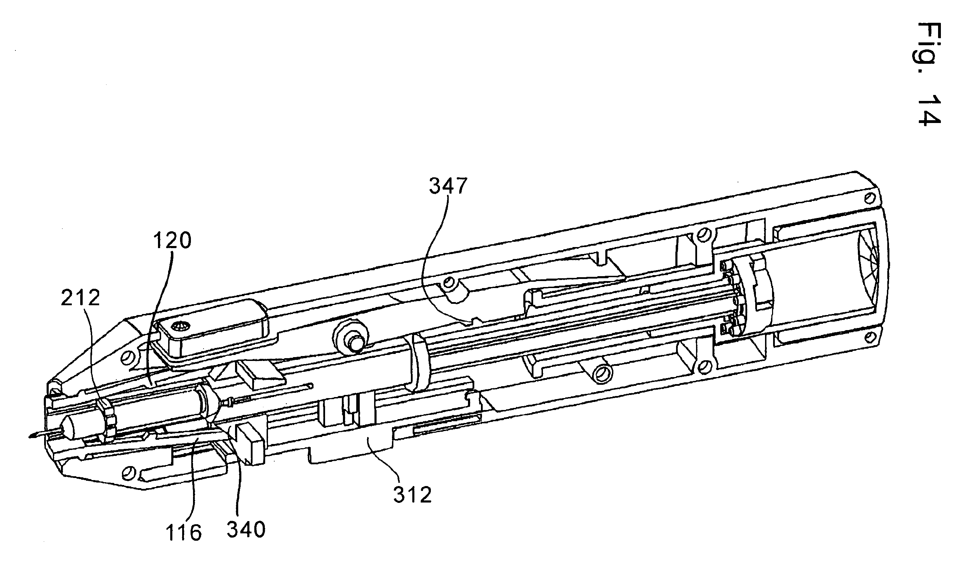

Figs. 13 and 14 schematically show the moment when the distal end portion 211 of the pricking member has just protruded from the front end opening 106 of the lancet case 102, similarly to Figs. 7 and 8. As seen from Fig. 14, the wings 116 are kept in the splayed out condition, the protruding portions 212 of the lancet body 204 have moved forward inside the stoppers 120 without being blocked by the stoppers 120.

It is noted that a return spring is provided between the protruding portion 324 of the plunger 310 and a front partition 344 which is provided inside the housing and around the plunger 310. This spring is such that it is compressed when the plunger 310 moves forward so as to launch the lancet. As a result, at the moment when the pricking is completed with the distal end portion 211 of the pricking member having protruded from the front end opening 106 of the lancet case102, or a time a little before or after such moment, the spring gets in the sufficiently compressed condition, and then it expands toward its original form, so that the lancet body is moved rearward after the pricking has been completed.

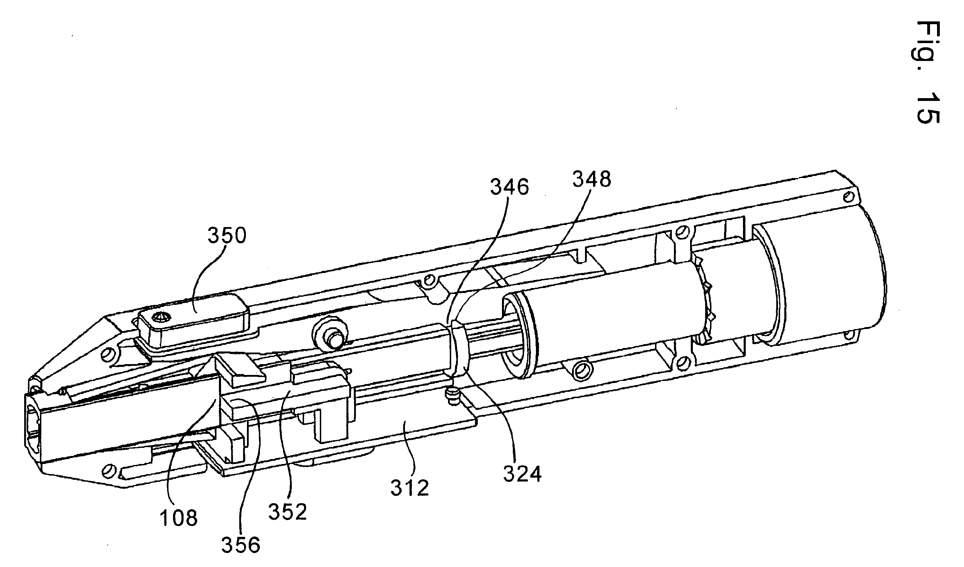

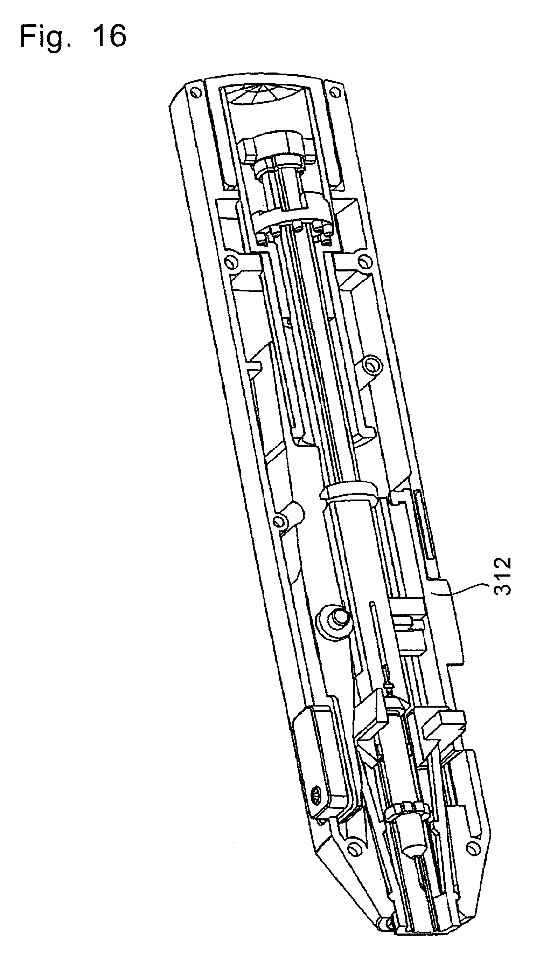

Figs. 15 and 16 schematically show the state where the lancet body 204 has been moved rearward from the state shown in Figs. 13 and 14, similarly to Figs. 7 and 8. As easily seen from the comparison of Fig. 12 with Fig. 16, the protruding portion 324 of the plunger has been in a fitting condition into a recess 348 located on the lower side of the trigger lever which recess is located behind a small protrusion 346 after having passed over the protrusion 346 by means of the expansion of the spring. By the provision of the protrusion 346 and the recess 348 in this way, the momentum of the plunger which is moving back is reduced, and the protruding portion 324 would not move forward over the protrusion 346 of the trigger lever even when the spring oscillation occurs in that the spring is compressed again after its expansion, which reduces a possibility of the second pricking.

In the state where the protruding portion 324 fits in the recess 348 which is located on the lower side of the trigger lever (in the shown embodiment, the side which is opposite to the side on which the push button is located) so to achieve the engagement condition, the protrusion 346 which is located in front of the recess 348 is configured such that such engagement is not removed even when a forward force is applied to the plunger. Specifically, a rear surface which defines the protrusion 346 (see Fig. 14) extends at right angle to the pricking direction or at an angle near such right angle while the front surface 349 which defines the protrusion 346 (see Fig. 13) extends obliquely with regard to the pricking direction to form a slope. When pressing the trigger button 350 for launching the lancet, the trigger lever rotates around the axis 326 and the protrusion 324 moves upward.

As a result, the protrusion 346 is not located on an orbit along which the protruding portion 324 of the plunger moves forward, so that it does not prevent the forward movement of the plunger. Then, the force applied to the trigger button is removed, the trigger lever returns to its original condition.

When such trigger lever is used, the engagement relationship between the protruding portion 324 and the protrusion 346 or the fitting relationship of the protruding portion 324 in the recess 348 is kept even though an ejector applies a forward force to the lancet case and such force is applied to the plunger. In order to ensure such relationship, the front surface 325 which define the protruding portion 324 extends at a right angle to the moving direction of the plunger or at an angle near such right angle while the rear surface 327 extends obliquely to form a slope.

By combining the front surface 325 at the right angle and the oblique rear surface 327 which define such protruding portion 324 with the oblique front surface 349 and the right angle rear surface 347 which define the protrusion 346 on the lower edge of the trigger lever as described above, it is possible that the protruding portion 324 of the plunger can pass over the protrusion 346 of the trigger lever so as to move rearward, but once having passed over the protrusion 346, the protruding portion 324 of the plunger cannot easily pass over the protrusion 346 of the trigger lever so as to move forward. In fact, in order to pass over the protrusion, at least a portion of the protruding portion or the protrusion has to substantially deform or break at least partly.

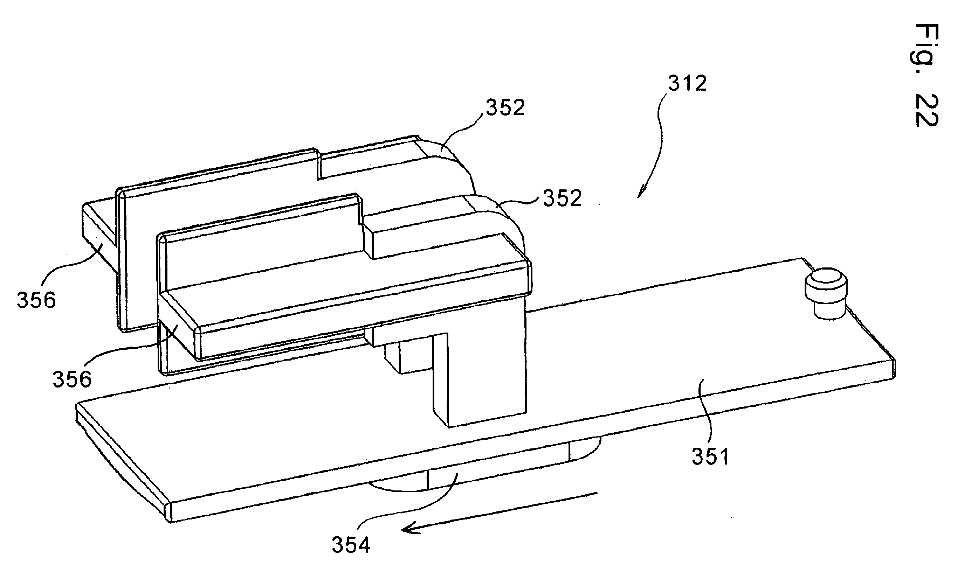

As shown in Figs. 7 to 16, the injector according to the present invention comprises an ejector 312 in its lower side. Such ejector is schematically shown in Fig. 22 in a perspective view. The ejector 312 comprises a base plate 351 and a pusher 352, and is disposed in a lower portion of the injector housing 309. The base plate 351 can be moved forward within the injector 300 by sliding the operation button 354 provided on the lower side of the base plate 351 from the outside of the injector in the direction of the arrow. For example, such a constitution may be employed as a guide channel 355 is provided on the inside of the injector housing and a rail is provided on the outside of the base plate for moving in the channel.

The plunger 352 is configured so that the front end portion 356 abuts against the rear end portion 108 of the lancet case 102 at the time when loading the lancet assembly 100 in the injector 300 is completed. As seen from the comparison of Figs. 7 and 8 with Figs. 9 to 16, the shown embodiment is configures as such. In other embodiment, the plunger 352 may be so positioned as to be spaced from the rear end portion 108 of the lancet case 102. It is noted that the pusher 352 is constituted so as to be capable of pushing forward the opposing side surfaces 128 of the case body 114 of the lancet case 102, more preferably only the surfaces 130 which include the rear end edges of the side surfaces where the wings are absent.

When the state as shown in Figs. 15 and 16 is achieved after the completion of the pricking, the rear end surfaces 130 of the lancet case 102 is pushed forward with the pusher 356 by sliding the button 354 of the ejector forward. When the force which pushes forward exceeds a threshold force which makes the bumps 112 of the lancet case pass over the protruding portions 304 at the front end opening 302 of the injector 300, the lancet case moves forward.

At this stage, the forward movement of the plunger is blocked by the protrusion 346 of the trigger lever and the recess 348 as described above. As a result, the force applied by the ejector is used first for moving the lancet case 102 forward, and then used for the disengagement of the rear end portion 202 of the lancet body 204 from the front end portion of the plunger as described below. The bump 218 of the rear end portion of the lancet body 204 (preferably a circumferential bump provided around the lancet body) in the lancet case 102 is pinched by the legs 320 and 322 of the front end portion of the plunger in the injector, so that the it is not moved forward.

When the lancet case 102 is moved forward as described, the rear end portions 120 of the wings 116 leave the wedge-shaped members 340, so that the wings 116 elastically deform toward their original forms. That is, the splayed out wings close so as to achieve the state as shown in Fig. 1. In this way, the stoppers 122 provided on the inside of the wings 116 again function as the stopper. That is, the protruding portions 212 of the lancet body 204 cannot pass forward over the stoppers 122 in the lancet case.

Since the forward movement of the lancet body 204 with the distal end portion 211 of the pricking member 210 is prevented by the stoppers 122, so that the distal end portion 211 of the pricking member can move forward only up to the position sufficiently away from the front end opening 106 of the lancet case 102. Of course, the protruding portions 212 cannot get out of the rear end opening of the lancet case 102. In this way, when the lancet case 102 moves forward and the wings 116 return to their original forms, the lancet body is kept in the lancet case 102 while the protruding distal end portions 211 is sufficiently behind the front end opening 106.

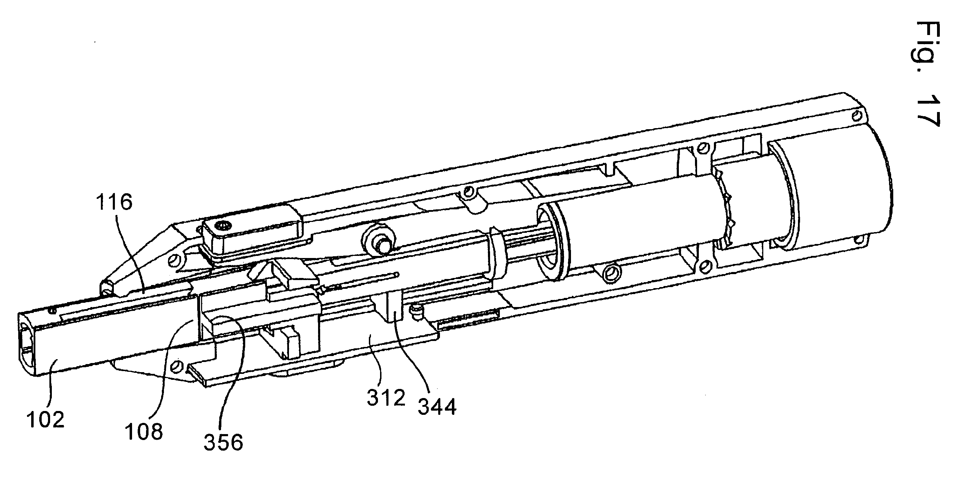

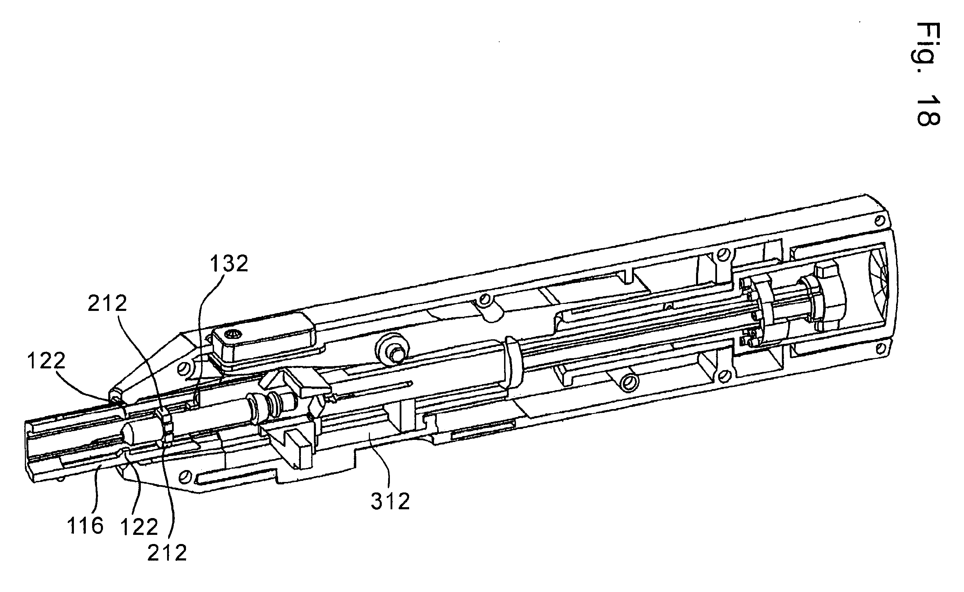

Figs. 17 and 18 schematically show the state where the lancet case 102 is moving forward by means of the ejector312, similarly to Figs. 7 and 8. As described above, the wings 116 have returned to their original forms. Therefore, the protruding portions 212 of the lancet body 204 prevented from their forward movement by the stoppers 122 of the wings 116, and also from their rearward movement by means of the wall 132 which defines the rear end opening 107 of the lancet case 102.

When the lancet case 102 is moved forward as shown, the protruding portions 212 of the lancet body 204 abut against the wall 132 which defines the rear end opening 107 of the lancet case 102. It is noted that the forward movement of the plunger is prevented by the trigger lever. In such stage, a force applied in order to intend to move the pusher 356 forward. When the force is applied forward, such force is transferred to the bump 218 of the lancet body which is hold, namely grasped by the legs 320 and 322 of the plunger 310, so that the bump 218 tries to splay the legs outward. When such force exceeds a certain threshold, the legs elastically splay outward, so that they release the bump 218 and the rear end portion of the lancet body 204 that they have grasped. It is noted that Figs. 15 to 18 show the state wherein the protruding portion 324 of the plunger is trapped in the recess 348 on the lower side of the trigger lever, and splaying out of the legs 320 and 322 is not prevented by the wall which defines the opening of the front partition 344 since the position of the front end portion 314 of the plunger is ahead of the front partition 344 of the housing.

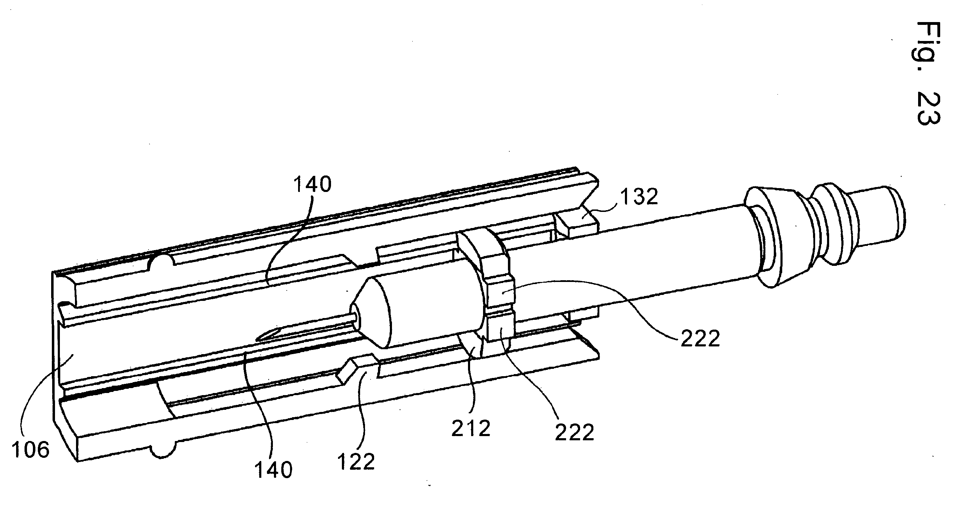

When the ejector 312 is moved further forward from the shown state, the lancet case 102, and therefore the lancet assembly is discharged from the injector 300 through its front end opening 302. The lancet assembly that has been discharged is comprises the lancet case 102 and the pricking member of which distal end portion 211 is exposed, and the state of such lancet assembly is shown in Fig. 23 similarly to Fig. 2. Easily seen, what is different from Fig. 2 is that the lancet cap 206 is not present in Fig. 23. As described above, the protruding portions 212 of the lancet body 206 are sandwiched by the stoppers 122 and the wall 132 of the rear end opening, and cannot move while getting over these members.

As easily seen from Fig. 23, even in the case wherein the lancet body 204 is moved most forward, the tip of the pricking member is sufficiently away from the front end opening 106 of the lancet case 102. Therefore, upon handling the lancet case 102 in the state as shown in Fig. 23, a risk is greatly reduced with respect to the exposed pricking member.

It is noted that the lancet body and he lancet case preferably have a means which makes the movement of the lancet body 204 smooth in the lancet case upon pricking. For example, as readily seen from Fig. 23, the lancet body 204 according to the present invention comprises other protruding portions 222, and the lancet case 102 has the rails 140 on the inside surface thereof so as to sandwich the protruding portions. By combining the protruding portions and the rails, a guide means may be formed which makes the movement of the lancet body 204 in the lancet case 102.

It is preferable that the lancet assembly and the injector are formed of a resin, and particularly resin molded articles except the pricking member and the springs. Such molded articles are preferable as to the function of the wings, the function of the plunger legs, and the relationships between the protruding portions 112 of the lancet case and the bumps at the front end opening of the injector in addition to the ease of production thereof.

In one embodiment of the injector according to the present invention, it comprises a pricking depth adjusting drum 360 disposed behind and adjacent to the rear partition 358 in the injector housing (see for example Fig. 7). A ring-shaped member 361 having an opening 363 is positioned in the inside of the front end of the drum 360 (these member are preferably connected together), and the rear end portion 362 of the plunger extends through the opening 363, and the rear end portion 362 has a hitting member 365. Further, the ring-shaped member is able to rotate around such plunger. Specifically, when a rotating knob 369 which is fitted in the rear end of the drum 360 is rotated, the drum 360 and the ring-shaped member 361 are able to be rotate around the plunger.

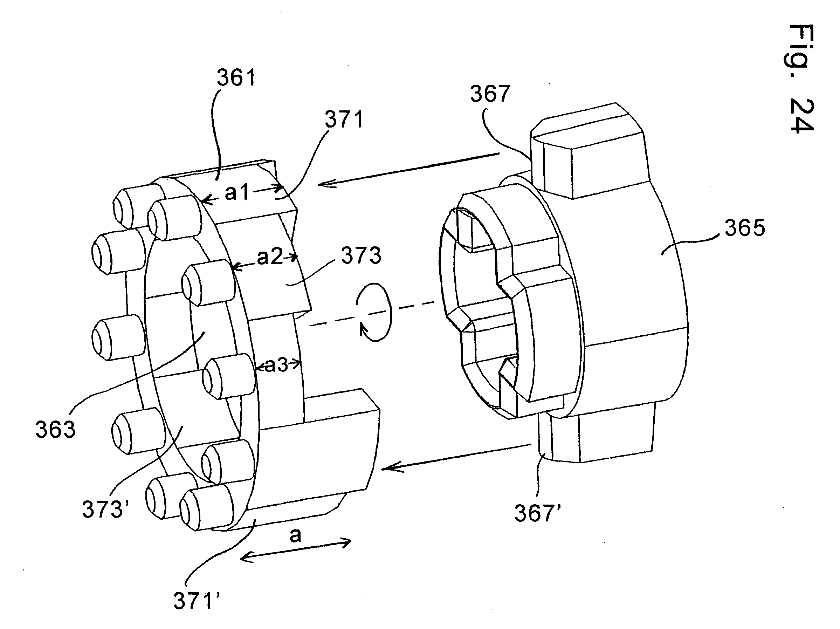

Fig. 24 schematically show the ring-shaped member located around the plunger and the hitting member 365 located at the rear end of the plunger in a perspective view with omitting the plunger (only of which extending direction is indicated with a broken line). The length (a) of the ring-shaped member 361 in the pricking direction changes along the circumferential direction thereof continuously or stepwise. In the embodiment illustrated, the length in the pricking direction changes stepwise for example as a1, a2, and a3. The hitting member 365 has on both sides thereof an upper hitting portion 367 and a lower hitting portion 367'. When the lancet is launched and the plunger moves forward, the hitting member 365 moves in the pricking direction as indicated by the arrow, and strikes the ring-shaped member.

In the embodiment shown in Fig. 37, for example, when the plunger moves forward, the hitting portion 367 strikes the step 371' of which length along the pricking direction is a1. That is, the hitting portion strikes one of the steps that constitute portions of the rear end surface of the ring-shaped member. When the knob 369 is turned a little clockwise (seeing from the left hand side in the drawing) as indicated by the arrow in the drawing in this state, the upper hitting portion 367 is moved to the near side, and the lower hitting portion 367' is moved to the further side.

As a result, the hitting portion 367 is allowed to strike the step 373 having a length a2 in the pricking direction, and the hitting member 367' is allowed to strike the step 373' having a length a2 in the pricking direction. As easily understood, as the lengths in the pricking direction of the ring-shaped member which the hitting portion strikes are different, for example the length in the pricking direction is different between step 371 and step 373, the distance over which the plunger can travel forward, namely the pricking depth changes.

In the case wherein at least one of the hitting member 365 and the ring-shaped member 361 is formed from an elasticity rich material such as a silicone, an urethane or the like so that it has an impact relieving property, the impact generated by these members when hitting can be mitigated. This provides an advantage of mitigating the impact which the user feels upon the pricking operation.

### Industrial Applicability

The lancet, the lancet case and the lancet assembly constituted from these members and the injector of the present invention provide a safer and convenient device.

## Claims

1. A lancet assembly comprising a lancet and a lancet case that houses a portion of the former, wherein
the lancet comprises a lancet body, a lancet cap and a pricking member made of a metal;
the lancet is a resin molded article in which the pricking member is disposed in the lancet body and the lancet cap while straddling over these members, the distal end portion of the pricking member is enclosed by the lancet cap, and the lancet cap and the lancet body are connected together through a weakened portion;
the lancet body has a protruding portion;
the lancet case comprises a case body, and wings disposed on both side surfaces of the case body; and
each wing has a stopper that protrudes inward between its front end portion and its rear end portion, the front end portion is connected to the case body and the rear end portion is left free and, as a result, when the stopper receives a force acting outward, the wing elastically splays outward with the force.

2. The lancet assembly according to claim 1 **characterized in that** the lancet cap comprises an outward protruding portion, preferably a protruding portion which extends circumferentially around the lancet cap.

3. The lancet assembly according to claim 1 or 2 **characterized in that** the stopper of the wing has a tapered off shape which extends inward toward the rear of the lancet case.

4. The lancet assembly according to any one of claims 1 to 3 **characterized in that** the lancet case comprises a front end opening and a rear end opening, and
when the lancet body is inserted in the lancet case through its front end opening, and moved in the lancet case rearward, the protruding portion of the lancet body abuts against a wall which defines the rear end opening of the lancet case.

5. The lancet assembly according to any one of claims 1 to 4 **characterized in that** the lancet case is engaged with an injector which launches the lancet, and includes a bump on the front end portion for hold the lancet assembly in the injector.

6. The lancet assembly according to any one of claims 1 to 5 **characterized in that** the lancet case is made by an injection molding of a resin, and as a result thereof, a front end portion of the wing is connected integrally to case body.

7. The lancet assembly according to any one of claims 1 to 6 **characterized in that** the lancet body comprises a rear end portion which fits in a front end portion of a plunger of an injector which launches the lancet, the rear end portion has a bump or a recess around its outside, and the bump or the recess fits in a recess or a bump which is complementarily provided in the front end portion of the plunger.

8. An injector which is used in combination with the lancet assembly according to any one of claims 1 to 7 so as to launch the lancet body with a distal end portion of the pricking member being exposed, **characterized in that**
the injector comprises therein a wing splaying member which expands each wing outward, and
when the lancet assembly is inserted rearward through a front end opening of the injector, each wing of the lancet case is elastically splayed outward by a member which expands the wing outward.

9. The injector according to claim 8 **characterized in that** the member which expands each wing outward is a pair of wedge-shaped members,
the wedge-shaped member is spaced from each other in a tapered off form so that a slope which is inclined toward the front of the injector, and
when the lancet assembly is inserted rearward through a front end opening of the injector, a rear end portion of each wing of the lancet case is located so as to position on a front end portion of the slope of the wedge-shaped member, and then when the insertion of the lancet assembly is continued, the rear end portion of the wing slides on the slope, so that wing is elastically splayed outward.

10. The injector according to claim 8 or 9 **characterized in that** it further comprises an ejector having a member which applies a forward force to the lancet case which is inserted.

11. The injector according to claim 10 **characterized in that** said member abuts against a rear end portion of the lancet case which is inserted.

12. The injector according to claim 10 or 11 **characterized in that** the ejector abuts against side surface of the lancet case which side surfaces are adjacent to side surfaces of the lancet case on which side surfaces the wings are present.

13. The injector according to any one of claims 8 to 12 **characterized in that** the plunger comprises a spring which is provided around the plunger and between a protruding portion provided around an intermediate portion of the plunger and a rear partition provided in an injector housing,
when a rear end portion of the lancet abuts against a front end of the plunger, the plunger is moved rearward so as to compress the spring and the plunger passes over a rear end of a trigger lever to which an inward force is applied, the protruding portion is engaged with the rear end of the trigger lever while the spring remains in the compressed condition, and
before the protruding portion is thus engaged, the rear end portion of the lancet body is grasped by the plunger by means of a rearward force which is applied to the rear end of the lancet body.

14. The injector according to any one of claims 8 to 13 **characterized in that** the trigger lever comprises on its lower side a protrusion and a recess which is adjacent to and behind the protrusion, and the protruding portion which is engaged with the rear end of the trigger lever fits in the recess of the trigger lever.

15. The injector according to any one of claims 8 to 14 **characterized in that**
the injector comprises a pricking depth adjusting drum disposed adjacent to and behind a rear partition in an injector housing,
a rear end portion of the plunger extends through the drum,
the drum has a ring-shaped member on the inside at the front end of the drum which member is capable of rotating around the plunger, and the length of the ring-shaped member in the pricking direction changes along the circumferential direction thereof continuously or stepwise,
the rear end of plunger has a hitting member that strikes the rear end surface in a portion along the circumferential direction of the ring-shaped member,
when the plunger moves forward for pricking, the hitting member on the rear end of plunger strikes a portion of the rear end surface of the ring-shaped member in the drum, and is therefore unable to move further forward, and
a portion of the rear end surface of the ring-shaped member as an object which the hitting member provided on the rear end of the plunger strikes is able to be changed by causing the ring-shaped member to rotate around the plunger by means of the drum rotation, so that the length between the rear partition of the housing of the injector and the rear end of the plunger is changed upon striking of the hitting member.

16. The injector according to claim 15 **characterized in that** the ring-shaped member has an impact relieving property.

17. A lancet which forms the lancet assembly according to any one of claims 1 to 7.

18. A lancet case which forms the lancet assembly according to any one of claims 1 to 7.

19. A kit of a lancet and a lancet case which are for the formation of the lancet assembly according to any one of claims 1 to 7.

20. A pricking device which is formed of the lancet assembly according to any one of claims 1 to 7 and the injector according to any one of claims 8 to 16.

21. A kit of a pricking device comprising the lancet assembly according to any one of claims 1 to 7 and the injector according to any one of claims 8 to 16.
